# EUROPEAN PATENT SPECIFICATION

(11) **EP 2 686 029 B1**
(45) Date of publication and mention of the grant of the patent: **29.11.2017**
(21) Application number: 12712731.4
(22) Date of filing: 12.03.2012
(51) Int. Cl.: A61L 29/16, A61L 29/08

(54) **METHOD FOR MAKING, BY A SOL-GEL PROCESS, CATHETERS INCLUDING ANTIBACTERIC COATINGS BASED ON USNIC ACID**
VERFAHREN ZUR HERSTELLUNG VON KATHETERN MIT ANTIBAKTERIELLEN BESCHICHTUNGEN AUF USNINSÄUREBASIS ANHAND EINES SOL-GEL-VERFAHRENS
PROCÉDÉ POUR LA FABRICATION, SELON UN PROCÉDÉ SOL-GEL, DE CATHÉTERS COMPORTANT DES REVÊTEMENTS ANTIBACTÉRIENS À BASE D'ACIDE USNIQUE

(30) Priority: 16.03.2011 IT MI20110426
(43) Date of publication of application: 22.01.2014
(73) Proprietor: Apharm S.r.l., 28041 Arona (NO) (IT)
(72) Inventor: POZZI, Paolo, I-28041 Arona, NO (IT); ZANASI, Tania, I-28041 Arona, NO (IT); TAURINO, Rosa, I-28041 Arona, NO (IT)
(74) Representative: Rastelli, Franco
(86) International application number: PCT/IB2012/000461
(87) International publication number: WO 2012/123803

(56) References cited:
- M. MARINI ET AL: "Antibacterial Activity of Plastic Coated with Silver-Dope Organic-Inorganic Hybrid Coatings Prepared by Sol-Gel Processes", BIOMACROMOLECULES, vol. 8, 3 March 2007 (2007-03-03), pages 1246-1254, XP002660482,
- BOGDAL D ET AL: "Microwave assisted synthesis, Crosslinking, and Processing of Polymeric Materials", ADVANCES IN POLYMER SCIENCE = FORTSCHRITTE DER HOCHPOLYMEREN-FORSCHUNG, SPRINGER, GERMANY, vol. 163, 1 January 2003 (2003-01-01), pages 193-263, XP008124400, ISSN: 0065-3195, DOI: 10.1007/B11051 [retrieved on 2003-08-21]
- I. FRANCOLINI ET AL: "Usnic acid, a Natural Antimicrobial Agent Able to Inhibit Bacterial Biofilm Formation on Polymer Surfaces", ANTIMICROBIAL AGENTS AND CHEMOTHERAPY, vol. 48, 1 November 2004 (2004-11-01), pages 4360-4365, XP002660483,

## Description

### BACKGROUND OF THE INVENTION

The present invention relates to a method for making, by a sol-gel process, silicone and PVC catheters and other medical implements coated by antibacteric coatings based on usnic acid. Catheters having antimicrobial polymeric coatings comprising usnic acid are known in the art (I. FRANCOLINI ET AL: "Usnic acid, a Natural Antimicrobial Agent Able to Inhibit Bacterial Biofilm Formation on Polymer Surfaces",ANTIMICROBIAL AGENTS AND CHEMOTHERAPY, vol. 48, 1 November 2004 (2004-11-01), pages 4360-4365). The invention has been specifically designed for coating silicone and PVC catheters to provide said catheters with a high antibacteric activity.

### SUMMARY OF THE INVENTION

Thus, the main object of the present invention has been that of providing a catheter antibacteric coating which, owing to its specifically designed structural features, is adapted to provide catheters and also medical use implements with very high reliable and safe antibacteric properties.

According to one aspect of the present invention, the above mentioned object, as well as further objects which will become more apparent hereinafter, have been achieved by an antibacteric coating for silicone and PVC catheters, as well as other medical implements and tools.

Further characteristics and advantages of the present invention will become more apparent hereinafter from the following disclosure of a preferred, though not exclusive, embodiment of the invention.

According to the present invention, silicone and PVC catheters and other medical implements have been made, by applying a hybrid organic-inorganic coating by means of a sol-gel applying process, in which, according to the main aspect of the invention, usnic acid has been included as an antibacteric agent.

As it is known, organic-inorganic hybrid coatings consist of a "monolithic grid or network" which is exclusively made by a sol-gel process, allowing to achieve two interconnected phases, the size of which varies from about 5 to 50 nanometers.

For the above reasons, the so-called "ceramic" materials may be considered as nanocompositions or compounds.

The above pattern provides said materials with structural features which are substantially different from those of the so-called "composite systems", in which the organic portion is added to the polymeric (organic) phase in a particle (fiber charged) form.

The organic-inorganic hybrid material or substance class according to the present invention is that in which the organic and inorganic phases are bound by strong ionic-covalent linkages or bindings.

The above mentioned "ceramics", which meet the above requirements, provide a class of materials which are very interesting from a scientific engineering standpoint, owing to their high interbinding degree and very good interfacing properties assured by strong chemical linkages between their matrix structure and reinforcement therefor.

Thus, by changing at will the ratios between the organic and inorganic phases, it is possible to provide materials having predominant features either of one or the other species.

Accordingly, it is possible to provide a high number of materials having intermediate features between those of ceramics and polymeric materials.

Considering a system which is richer in the organic component, the silica nano-domains will be formed by the well known sol-gel process, and will represent nodes of a 3D grid being formed during the gel forming phase.

The reaction components allowing to achieve the above mentioned ceramic materials consist of soluble metal alkoxide (usually based on Si, Ti, Al or Zr) and an oligomer or polymer, optionally including an alkoxysilane functionality.

If they are present, then it is possible to functionalize the end oligomer hydroxyl or amine groups by causing them to react with the reactive organic groups of a silane binding agent.

For making the antibacteric catheter coatings according to the present invention, a sol-gel method or process has been used, in which the gel is a rigid and disordered cross-linked grid formed by interconnecting polymeric chains with an average length in the order of micrometers, with pores having a pore size smaller than 1 micrometer.

The silica gel is based on a tetrahedral group structure (SiO₄)ₙ, the tetrahedral groups of which are intercoupled by Si-O-Si bridges.

As it is known, a sol consists of a colloidal particle dispersion in a liquid, the colloidal particles of which consist of solid particles having a diameter of the order of 1-100 nanometers.

The sol-gel methods comprise processes wherein a solution or sol provides a sol-gel transition causing the solution to be transformed into a rigid and porous mass.

A silica gel may be prepared by polymerizing a silicon alkoxyde sol-gel composition.

Applicant has chosen to use the above mentioned coating method at first because of its low processing temperatures, since Usnic Acid (that is the inventive antibacteric agent) has a comparatively poor thermal resistance, with a maximum processing temperature of 60°C.

More specifically, by means of the above sol-gel processes, it is possible to form an inorganic network at a temperature near to the room temperature.

A second main reason of using the sol-gel method for providing the inventive antibacteric coating is the fact that this method allows to increase the interface adhesion between the organic phases, while facilitating an intimate mutual interlacing to provide an end product having high purity and homogeneity properties.

Thus, it is possible to provide catheter and other medical implement coatings having very thin thickness and with structural properties specifically tailored for meeting a lot of requirements, since an addition of substances within the coating network provides the coating with target functions, such as a high flame resistance, antibacteric, scratch resistance properties, and so on.

In this connection Applicant points out that attempts to make antibacteric coatings by using silver or ammonium salts as antibacteric agent had already been made.

However, the above silver or ammonium salt based coatings were affected by the problem of a short duration of the antibacteric effect, with a poor spreading of the antibacteric agent on the coating outside.

Yet another drawback of the above silver and ammonium salt based coatings was their very high cost.

Thus, a further aim of the present invention is that of overcoming the above drawbacks of prior antibacteric coatings including silver or ammonium salts compounds.

A further object of the present invention has been that of providing antibacteric compositions easily applicable to different supports, such as catheters and other medical implements, by the same applying or processing technique.

Another object of the present invention has been that of providing a method adapted to be easily carried out, in a very safe and efficient manner, and providing low cost antibacteric coatings for silicone and PVC catheters.

According to the invention, the above aim and objects, as well as further objects, have been achieved by a method as claimed in claim 1, which is further characterized in that it comprises a first step of dissolving the organic phase (the polymer) both from a functional standpoint and non-functional standpoint, under stirring and a slight heating, in a suitable solvent, thereby providing a diluted mixture to which the inorganic grid forming agent (the alkoxide) is then added in different organic/inorganic ratios.

By holding the mixture under stirring, the usnic acid antibiotic agent is added thereto, thereby embedding said usnic acid into the grid forming phase.

In this process, to properly start the grid gel forming phase, a solution of H₂O, ethanol and HCl is added.

Then, catheter specimens are coated by immersing them into the thus made solution or by spraying or bathing said specimens with the solution.

Then, as an end step of said method, said gel grid is processed by a microwave thermal heating process.

Further characteristics and advantages of the method according to the present invention will become more apparent from the following detailed disclosure of a preferred, though not exclusive, embodiment of a method for making, by a sol-gel process, antibacteric usnic acid coated catheters.

More specifically, the method according to the present invention comprises a first step of dissolving an organic precursor (organic phase) in a suitable solvent under stirring and heating at a temperature from 25°C to 70°C at a concentration from 10% to 50% by weight.

Then, said method comprises a further step of adding the inorganic gel grid (alkoxide) forming agent with organic/inorganic ratios of 1:1, 1:2, 2:1, while holding under stirring.

Then, said antibiotic usnic acid solution is embedded in the thus formed gel grid in a rate from 0.1% to 10%.

For starting the gel grid forming step, a solution of H₂O/ethanol/HCl or other acids and alcohols, such as sulphuric or hydrofluoric acids and alcohols, such as methanol or butanol, is further added to provide a coating mixture which is then applied to a surface to be processed by immersing, or spraying or bathing, the application system and time affecting the thickness of the applied coating.

Then, a final crosslinking step is carried out by a microwave thermal process for a time from two seconds to a few hours, at temperatures from room temperature to 60°C and with a microwave power from 300 to 900 W.

On the thus made catheters the following tests have been carried out together with a screening for evaluating the antibacteric activity of said catheters according to the ASTM 2180-07 standard.

More specifically, yellow catheter specimens or samples and clear catheter specimens have been screened, having a length of about 2 cm, and processed by usnic acid according to four different processing modes, as well as yellow and clear catheter specimens not processed by usnic acid (as a negative control).

In the above tests the following microorganism have been used:

| **Microorganism** | **Code** |
|---|---|
| Staphilococcus aureus | ATCC 6538 |
| Eneterococcus hirae | ATCC 10541 |
| Escherichia coli | ATCC 10536 |
| Proteus mirabilis | ATCC 10005 |

Both the yellow and clear catheter specimens have been subjected to the following treatments:

| **Treatment** | **Code** |
|---|---|
| 1 | Thermal PV-OH 8-88 APTES |
| 2 | PV-OH 8-88 APTES MW |
| 3 | Thermal APTES |
| 4 | APTES MW |

The rate reductions achieved with respect to the negative control, after a contact time of 24 hours, are shown in the following Tables:

### 1. YELLOW CATHETERS

| **Microorganism** | **Treatment 1** | **Treatment 2** | **Treatment 3** | **Treatment 4** |
|---|---|---|---|---|
| | PV-OH 8-88 + Thermal APTES | PV-OH 8-88 + APTES MW | Thermal APTES | APTES MW |
| S. aureus | 98.485 | >99.997 | 99.364 | >99.997 |
| E. hiare | 98.423 | >99.996 | 99.000 | >99.996 |
| E coli | >99.994 | >99.994 | 97.722 | >99.994 |
| P. mirabilis | >99.997 | >99.997 | 98.759 | >99.997 |

### 2. CLEAR CATHETERS

| **Microorganism** | **Treatment 1** | **Treatment 2** | **Treatment 3** | **Treatment 4** |
|---|---|---|---|---|
| | PV-OH 8-88 + Thermal APTES | PV-OH 8-88 + APTES MW | Thermal APTES | APTES MW |
| S. aureus | 97.879 | 99.994 | 98.939 | 99.988 |
| E. hiare | 98.269 | 99.981 | 98.423 | 99.981 |
| E coli | >99.994 | >99.994 | 95.000 | 99.950 |
| P. mirabilis | >99.997 | >99.997 | 97.828 | 99.986 |

Based on the above screening results, it should be apparent that the screened USNIC ACID TREATED CATHETERS were provided with a prominent antibacteric activity, causing, after the mentioned contact time, a reduction of the microbic rate or vitality greater than 1 Log (% reduction > 90%) with respect to the negative control.

### RESULTS

The rate reductions achieved with respect to the negative control after a contact time of 24 hours are shown in the following Tables:

### 1. YELLOW CATHETERS

| **Microorganism** | **Treatment 1** | **Treatment 2** | **Treatment 3** | **Treatment 4** |
|---|---|---|---|---|
| | PV-OH 8-88 + Thermal APTES | PV-OH 8-88 + APTES MW | Thermal APTES | APTES MW |
| S. aureus | 98.485 | >99.997 | 99.364 | >99.997 |
| E. hiare | 98.423 | >99.996 | 99.000 | >99.996 |
| E coli | >99.994 | >99.994 | 97.722 | >99.994 |
| P. mirabilis | >99.997 | >99.997 | 98.759 | >99.997 |

### 2. CLEAR CATHETERS

| **Microorganism** | **Treatment 1** | **Treatment 2** | **Treatment 3** | **Treatment 4** |
|---|---|---|---|---|
| | PV-OH 8-88 + Thermal APTES | PV-OH 8-88 + APTES MW | Thermal APTES | APTES MW |
| S. aureus | 97.879 | 99.994 | 98.939 | 99.988 |
| E. hiare | 98.269 | 99.981 | 98.423 | 99.981 |
| E coli | >99.994 | >99.994 | 95.000 | 99.950 |
| P. mirabilis | >99.997 | >99.997 | 97.828 | 99.986 |

No offset during the screening tests has been detected.

Thus, from the above screening test results it clearly appears that the ACID USNIC TREATED CATHETERS according to the present invention have a prominent antibacteric activity since they provide, after the indicated contact time, a microbic rate reduction larger than 1 Log (% reduction >90%) with respect to the negative controls.

The most efficient treatments were treatment 2 (PV-OH 8-88 + APTES MW) and treatment 4 (APTES MW), both for usnic acid treated yellow catheters and for usnic acid treated clear or transparent catheters; treatment 2 and treatment 4 were found as the most efficient on usnic acid treated yellow catheters.

It has been found that the invention fully achieves the intended aim and objects.

## Claims

1. A method for making an antibacteric coating for silicone and PVC catheters and medical implements, **characterized in that** said method comprises the steps of providing a hybrid organic-inorganic composition made by a sol-gel process, including in said composition an antibacteric agent consisting of usnic acid to provide an antibacteric coating composition and applying said antibacteric coating composition to said catheter by a microwave crosslinking process.

2. A method, according to claim 1, **characterized in that**, in said sol-gel step, said gel comprises a rigid and irregular gel grid, formed by interconnected polymeric chains having an average chain length in the order of micrometers, with pores having a size smaller than one micrometer.

3. A method, according to claim 1, **characterized in that** said method comprises the step of using a silica gel, based on a tetrahedron group structure (SiO₄), including tetrahedron groups interconnected by Si-O-Si interconnecting bridges.

4. A method, according to claim 3, **characterized in that** said silica gel is prepared by polymerizing silicon alkoxide sols-gels.

5. A method, according to claim 1, **characterized in that** said method comprises the following steps: a first dissolving step of dissolving the organic or polymeric phase, either functionalized or not, under stirring and a slight heating in a diluting and dissolving solvent, adding an inorganic alkoxide grid forming agent, in different organic/inorganic ratios, and, while holding under stirring, further adding an antibiotic usnic acid agent, so as to embed said antibiotic agent in said gel grid.

6. A method, according to claim 1, **characterized in that** the grid gel forming step is started by adding a solution of H₂O, ethanol and HCl.

7. A method, according to claim 1, **characterized in that** said method further comprises a step of coating catheter specimens by immersing said catheter specimens into a prepared solution or by spraying or bathing said catheter specimens by said prepared solution.

8. A method, according to claim 1, **characterized in that** said method comprises a dissolving step of dissolving an organic phase precursor in a solvent under stirring and heating from 25°C to 70°C at a concentration from 10% to 50% by weight.

9. A method, according to claim 1, **characterized in that** said method comprises the step of adding the inorganic gel grid alkoxide forming agent with organic/inorganic ratios of 1:1, 1:2, 2:1, while holding under stirring.

10. A method, according to claim 1, **characterized in that** said antibiotic usnic acid agent solution is so added as to embed said antibiotic agent solution in the formed gel grid in a rate from 0.1% to 10%.

11. A method, according to claim 1, **characterized in that** said method further comprises the step of starting the gel grid forming step, by adding a solution of H₂O/ethanol/HCl or other acids and alcohols, such as sulphuric or hydrofluoric acid and alcohols, such as methanol or butanol.

12. A method, according to claim 1, **characterized in that** said coating is applied to a catheter surface by immersing, spraying or bathing.

13. A method, according to claim 1, **characterized in that** said method comprises a crosslinking step carried out by a microwave thermal process for a time from two seconds to several hours, at temperatures from a room temperature to 60°C, with a microwave power from 300 to 900 Watts.

## Patentansprüche

1. Verfahren zum Herstellen einer antibakteriellen Beschichtung für Silikon- und PVC-Katheter und medizinische Geräte, **dadurch gekennzeichnet, dass** das Verfahren die folgenden Schritte umfasst: Bereitstellen einer hybriden organisch-anorganischen Zusammensetzung, die durch einen Sol-Gel-Prozess hergestellt wird, wobei in der Zusammensetzung ein antibakterielles Mittel enthalten ist, das aus Usninsäure besteht, um eine antibakterielle Beschichtungszusammensetzung bereitzustellen, und Aufbringen der antibakteriellen Beschichtungszusammensetzung auf den Katheter durch einen Mikrowellen-Vernetzungsprozess.

2. Verfahren nach Anspruch 1, **dadurch gekennzeichnet, dass** das Gel in dem Sol-Gel-Schritt ein starres und unregelmäßiges Gelgitter umfasst, das durch miteinander verbundene Polymerketten, die eine durchschnittliche Kettenlänge in der Größenordnung von Mikrometern aufweisen, mit Poren gebildet ist, die eine Größe von weniger als einem Mikrometer aufweisen.

3. Verfahren nach Anspruch 1, **dadurch gekennzeichnet, dass** das Verfahren den Schritt des Verwendens eines Silikagels basierend auf einer Tetraedergruppenstruktur (SiO₄), einschließlich Tetraedergruppen, die durch Si-O-Si-Verbindungsbrücken miteinander verbunden sind, umfasst.

4. Verfahren nach Anspruch 3, **dadurch gekennzeichnet, dass** das Silicagel durch Polymerisieren von Siliciumalkoxid-Sol-Gelen hergestellt wird.

5. Verfahren nach Anspruch 1, **dadurch gekennzeichnet, dass** das Verfahren die folgenden Schritte umfasst: einen ersten Auflösungsschritt des Auflösens der organischen oder polymeren Phase, die entweder funktionalisiert ist oder nicht, unter Rühren und leichtem Erwärmen in einem Verdünnungs- und Lösungsmittel, Zugeben eines anorganischen Alkoxid-Gitterbildners in verschiedenen organischen/anorganischen Verhältnissen und, während das Rühren fortgesetzt wird, weiteres Zugeben eines Usninsäure-Antibiotikums, um das Antibiotikum in das Gelgitter einzubetten.

6. Verfahren nach Anspruch 1, **dadurch gekennzeichnet, dass** der Gittergelbildungsschritt durch Zugeben einer Lösung von H₂O, Ethanol und HCl gestartet wird.

7. Verfahren nach Anspruch 1, **dadurch gekennzeichnet, dass** das Verfahren ferner einen Schritt des Beschichtens von Katheterproben durch Eintauchen der Katheterproben in eine hergestellte Lösung oder durch Aufsprühen oder Baden der Katheterproben durch die hergestellte Lösung umfasst.

8. Verfahren nach Anspruch 1, **dadurch gekennzeichnet, dass** das Verfahren einen Auflösungsschritt des Auflösens eines Vorläufers einer organischen Phase in einem Lösungsmittel unter Rühren und Erwärmen von 25 °C auf 70 °C bei einer Konzentration von 10 Gew.-% bis 50 Gew.-% umfasst.

9. Verfahren nach Anspruch 1, **dadurch gekennzeichnet, dass** das Verfahren während des Haltens unter Rühren den Schritt des Zugeben des anorganischen Alkoxid-Gelgitterbildners mit organischen/anorganischen Verhältnissen von 1:1, 1:2, 2:1 umfasst.

10. Verfahren nach Anspruch 1, **dadurch gekennzeichnet, dass** die Usninsäure-Antibiotikum-Lösung derart zugegeben wird, dass die Antibiotikum-Lösung in dem gebildeten Gelgitter in einer Menge von 0,1 % bis 10 % eingebettet wird.

11. Verfahren nach Anspruch 1, **dadurch gekennzeichnet, dass** das Verfahren ferner den Schritt des Startens des Gelgitterbildungsschritts durch Zugeben einer Lösung von H₂O/Ethanol/HCl oder anderer Säuren und Alkohole wie Schwefel- oder Fluorwasserstoffsäure und Alkoholen wie Methanol oder Butanol umfasst.

12. Verfahren nach Anspruch 1, **dadurch gekennzeichnet, dass** die Beschichtung durch Eintauchen, Aufsprühen oder Baden auf eine Katheteroberfläche aufgebracht wird.

13. Verfahren nach Anspruch 1, **dadurch gekennzeichnet, dass** das Verfahren einen Vernetzungsschritt umfasst, der durch einen thermischen Mikrowellenprozess für eine Zeit von zwei Sekunden bis zu mehreren Stunden bei Temperaturen von Raumtemperatur bis 60 °C mit einer Mikrowellenleistung von 300 bis 900 Watt ausgeführt wird.

## Revendications

1. Procédé de fabrication d'un revêtement antibactérien pour des cathéters et accessoires médicaux en silicone et PVC, **caractérisé en ce que** ledit procédé comprend les étapes de fourniture d'une composition organique-inorganique hybride fabriquée par un procédé sol-gel, comprenant dans ladite composition un agent antibactérien constitué d'acide usnique pour fournir une composition de revêtement antibactérienne et l'application de ladite composition de revêtement antibactérienne au dit cathéter par un procédé de réticulation par micro-ondes.

2. Procédé, selon la revendication 1, **caractérisé en ce que**, dans ladite étape sol-gel, ledit gel comprend un réseau de gel rigide et irrégulier, formé par des chaînes polymères interconnectées ayant une longueur de chaîne moyenne de l'ordre des micromètres, avec des pores ayant une taille inférieure à un micromètre.

3. Procédé, selon la revendication 1, **caractérisé en ce que** ledit procédé comprend l'étape d'utilisation d'un gel de silice, basé sur une structure de groupe tétraédrique (SiO₄), comprenant des groupes tétraédriques interconnectés par des liaisons d'interconnexion Si-O-Si.

4. Procédé, selon la revendication 3, **caractérisé en ce que** ledit gel de silice est préparé par polymérisation de sols-gels d'alcoxyde de silicium.

5. Procédé, selon la revendication 1, **caractérisé en ce que** ledit procédé comprend les étapes suivantes : une première étape de dissolution de la phase organique ou polymère, fonctionnalisée ou non, sous agitation et un léger chauffage dans un solvant de dilution et de dissolution, l'ajout d'un agent de formation de réseau alcoxyde inorganique, en différents rapport organique/inorganique, et, tout en maintenant sous agitation, l'ajout ultérieur d'un agent antibiotique acide usnique, pour intégrer ledit agent antibiotique dans ledit réseau de gel.

6. Procédé, selon la revendication 1, **caractérisé en ce que** l'étape de formation de gel de réseau débute par ajout d'une solution de H₂O, d'éthanol et de HCl.

7. Procédé, selon la revendication 1, **caractérisé en ce que** ledit procédé comprend en outre une étape de revêtement de spécimens de cathéters par immersion desdits spécimens de cathéter dans une solution préparée ou par pulvérisation ou par trempage desdits spécimens de cathéter par ladite solution préparée.

8. Procédé, selon la revendication 1, **caractérisé en ce que** ledit procédé comprend une étape de dissolution consistant à dissoudre un précurseur de phase organique dans un solvant sous agitation et chauffage de 25 °C à 70 °C à une concentration comprise entre 10 % et 50 % en poids.

9. Procédé, selon la revendication 1, **caractérisé en ce que** ledit procédé comprend l'étape d'ajout de l'agent alcoxyde de formation de réseau de gel inorganique à des rapports organiques/inorganiques de 1/1, 1/2, 2/1, tout en maintenant sous agitation.

10. Procédé, selon la revendication 1, **caractérisé en ce que** ladite solution d'agent antibiotique acide usnique est ajoutée pour intégrer ladite solution d'agent antibiotique dans le réseau de gel formé à un taux compris entre 0,1 % et 10 %.

11. Procédé, selon la revendication 1, **caractérisé en ce que** ledit procédé comprend en outre l'étape consistant à débuter l'étape de formation de réseau de gel, par l'ajout d'une solution d'H₂O/éthanol/HCl ou autres acides et alcools, tels que l'acide sulfurique ou fluorhydrique et des alcools, tels que le méthanol ou le butanol.

12. Procédé, selon la revendication 1, **caractérisé en ce que** ledit revêtement est appliqué à une surface de cathéter par immersion, pulvérisation ou trempage.

13. Procédé, selon la revendication 1, **caractérisé en ce que** ledit procédé comprend une étape de réticulation réalisée par un processus thermique par micro-ondes pendant une durée de deux secondes à plusieurs heures, à des températures comprises entre température ambiante et 60 °C, avec une puissance micro-onde de 300 à 900 Watts.
